Europäisches Patentamt

European Patent Office

Office européen des brevets

(11) Numéro de publication: **0 027 768**
**B1**

(12) **FASCICULE DE BREVET EUROPEEN**

(45) Date de publication du fascicule du brevet:
**10.08.83**

(51) Int. Cl.³: **A 61 K 9/18**, B 01 J 39/18,
C 07 D 401/06

(21) Numéro de dépôt: **80401486.8**

(22) Date de dépôt: **20.10.80**

(54) **Nouvelles résines échangeuses d'ions du type acide, leur préparation, leur application comme médicaments et les compositions les renfermant.**

(30) Priorité: **23.10.79 FR 7926221**
**31.01.80 JP 9464/80**

(43) Date de publication de la demande:
**29.04.81 Bulletin 81/17**

(45) Mention de la délivrance du brevet:
**10.08.83 Bulletin 83/32**

(84) Etats contractants désignés:
**BE CH DE FR GB IT LI NL**

(56) Documents cités:
**FR-A-1 550 956**
**FR-A-2 339 402**
**US-E-26 833**

«**Chemical Abstracts**»,vol. 81, 23 décembre 1974, p. 12, abrégé 163268e Columbus, Ohio, USA; Yvore P., «**Efficiency of stenorol in the chemoprophylaxis of avian coccidiosis**».
«**Remington's Pharmaceutical Sciences**» (1975), pp. 1625, 1626.

**Le dossier contient des informations techniques présentées postérieurement au dépôt de la demande et ne figurant pas dans le présent fascicule.**

(73) Titulaire: **ROUSSEL-UCLAF, 35, boulevard des Invalides, F-75007 Paris (FR)**

(72) Inventeur: **Martel, Jacques, 15, rue Douvillez, F-93140 Bondy (FR)**
Inventeur: **Tessier, Jean, 30, rue Jean Moulin, F-94300 Vincennes (FR)**
Inventeur: **Girault, Pierre, 4, rue des Abbesses, F-75018 Paris (FR)**
Inventeur: **Grandadam, Jean, 4, rue Lhomme, F-94100 Saint-Maur-des-Fosses (FR)**

(74) Mandataire: **Petranker, Léon et al, boîte postale no 9 102, route de Noisy, F-93230 Romainville (FR)**

ACTORUM AG

# Nouvelles résines échangeuses d'ions du type acide, leur préparation, leur application comme médicaments et les compositions les renfermant

La présente invention concerne de nouvelles résines échangeuses d'ions du type acide, leur préparation, leur application comme médicaments et les compositions les renfermant.

L'invention a pour objet les résines échangeuses d'ions du type acide dont les groupements acides sont au moins partiellement salifiés par la 7-bromo 6-chlorofébrifugine.

Par 7-bromo 6-chlorofébrifugine, on entend le composé racémique tel qu'il est décrit dans le brevet FR-A No 1550956 ou l'un de ses isomères actifs, l'isomère dextrogyre décrit dans la demande de brevet FR-A No 2339402.

Les résines de l'invention sont tout à fait inattendues. Il n'était pas évident à priori de préparer des résines d'une molécule comme la 7-bromo 6-chlorofébrifugine.

Par résine acide, on entend notamment les résines à groupements sulfoniques, phosphoniques et carboxyliques.

L'invention a plus particulièrement pour objet les résines sulfoniques.

Toutes les résines sulfoniques acides peuvent convenir pour préparer les produits de l'invention; on peut utiliser, par exemple, des polymérisats sulfonés de composés polyvinylaryliques, tels que décrits dans le brevet US-A No 2366007, et notamment des polymérisats sulfonés de styrène-divinylbenzène.

On peut également utiliser des résines à base de polymérisats acide méthacrylique/divinylbenzène.

L'invention n'est pas limitée à une catégorie de résines de granulométrie ou de réticulation donnée.

On peut utiliser tout particulièrement les résines Amberlite du type de celle utilisée dans la partie expérimentale exposée ci-après, mais il va de soi que les autres résines du commerce à groupements acides forts peuvent convenir pour préparer les produits de l'invention.

L'invention a plus particulièrement pour objet les résines définies précédemment, caractérisées en ce que les groupements acides ne sont que partiellement salifiés par la 7-bromo 6-chlorofébrifugine, notamment celles caractérisées en ce que les groupements acides non salifiés par la 7-bromo 6-chlorofébrifugine sont salifiés par un métal compatible avec les impératifs de la nutrition animale, par exemple les métaux alcalins, les alcalino-terreux, le magnésium, le manganèse, le fer, le cuivre, le zinc.

L'invention a tout particulièrement pour objet les résines définies précédemment, caractérisées en ce que les groupements acides non salifiés par la 7-bromo 6-chlorofébrifugine sont salifiés par un métal alcalin ou alcalino-terreux.

L'invention a également pour objet les résines définies précédemment, caractérisées en ce que les groupements acides non salifiés par la 7-bromo 6-chlorofébrifugine sont salifiés par du manganèse, du magnésium, du fer, du cuivre, ou du zinc.

Par métal alcalin, on entend de préférence le sodium et le potassium.

Par métal alcalino-terreux, on entend de préférence le calcium.

L'invention a ainsi tout spécialement pour objet les résines définies précédemment, caractérisées en ce que les groupements acides non salifiés par la 7-bromo 6-chlorofébrifugine sont salifiés par du sodium ou par du calcium.

L'invention a notamment pour objet les résines définies précédemment, caractérisées en ce qu'elles renferment de 1 à 20% et de préférence de 5 à 10% en poids de 7-bromo 6-chlorofébrifugine.

L'invention a tout spécialement pour objet les résines définies précédemment, caractérisées en ce que la 7-bromo 6-chlorofébrifugine utilisée est le produit racémique.

L'invention a tout spécialement pour objet les résines dont la préparation est donnée ci-après dans la partie expérimentale.

Les produits de l'invention présentent d'intéressantes propriétés pharmacologiques; ils présentent toutes les activités de la 7-bromo 6-chlorofébrifugine, et notamment une très bonne activité coccidiostatique, une très bonne activité antileucocytozoonose ainsi qu'une faible toxicité.

L'invention a donc pour objet les produits de l'invention à titre de médicaments.

Les médicaments de l'invention peuvent être utilisés en particulier chez la volaille, les ovins et les bovins pour lutter contre la coccidiose et, chez la volaille, pour lutter contre le leucocytozoonose.

L'invention a également pour objet les compositions pharmaceutiques renfermant comme principe actif au moins un médicament défini précédemment.

Ces compositions sont utilisées, de préférence, par voie orale; elles peuvent être présentées sous forme de poudres, de comprimés, de comprimés enrobés, de cachets, de capsules, de granulés, d'émulsions ou de sirops.

Le ou les principes actifs peuvent être incorporés à des excipients habituellement employés dans ces compositions telles que le talc, la gomme arabique, le lactose, l'amidon, le stéarate de magnésium, les véhicules aqueux ou non, les corps gras d'origine animale ou végétale, les dérivés paraffiniques, les glycols, les divers agents mouillants, dispersants ou émulsifiants, les conservateurs. Elles sont préparées selon les méthodes usuelles.

La posologie utile est habituellement comprise entre 5 et 100 parties des produits de l'invention par million de la quantité de nourriture ingérée par l'animal. La posologie est donc comprise, par exemple, entre 100 et 20 000 $\gamma$ de produit actif pour un poulet mangeant entre 20 et 200 g de nourriture par jour.

Les produits de l'invention sont très souvent incorporés aux compositions alimentaires qui renferment alors au moins un produit défini ci-dessus associé à un mélange nutritif adapté à l'alimentation animale. Le mélange nutritif peut varier selon l'espèce animale: il peut renfermer des céréales, des sucres et graines, des tourteaux de soja, d'arachide et de tournesol, des farines d'origine animale, par exemple des farines de poisson, des acides aminés de synthèse, des sels minéraux, des vitamines, des antioxydants.

L'invention a donc ainsi également pour objet les compositions alimentaires définies ci-dessus.

L'invention a également pour objet un procédé de préparation des résines définies ci-dessus, caractérisé en ce que l'on salifie au moins partiellement une résine par la 7-bromo 6-chlorofébrifugine et obtient ainsi le produit recherché.

L'invention a notamment pour objet un procédé, caractérisé en ce que l'on soumet une résine acide dont les groupements acides sont salifiés par un métal alcalin, à l'action d'un sel acide de 7-bromo 6-chlorofébrifugine, pour obtenir un sel mixte de métal alcalin et de 7-bromo 6-chlorofébrifugine de la résine acide, que l'on soumet, si désiré, à l'action d'un sel soluble de métal non alcalin, pour obtenir un sel mixte de la 7-bromo 6-chlorofébrifugine et de métal non alcalin de la résine acide.

Par métal non alcalin, on entend de préférence les métaux alcalino-terreux, le manganèse, le magnésium, le fer, le cuivre ou le zinc.

Les nouvelles résines selon l'invention peuvent notamment être obtenues par un procédé selon lequel l'on additionne une résine de type acide éventuellement salifiée à une solution de 7-bromo 6-chlorofébrifugine ou d'un sel de 7-bromo 6-chlorofébrifugine avec un acide ou l'on fait passer ladite solution sur une colonne renfermant la résine. Ainsi, en modifiant le type ou la quantité de résine, on peut ajuster la proportion de 7-bromo 6-chlorofébrifugine dans le sel obtenu à la valeur que l'on souhaite obtenir, pour autant, bien entendu, que cette valeur se situe en deçà de la capacité d'échange de la résine utilisée.

Dans un mode d'exécution préférentiel de ce procédé, le solvant de la 7-bromo 6-chlorofébrifugine utilisé peut être l'eau ou des solvants organiques, par exemple le méthanol, l'éthanol, l'acétone ou encore le chloroforme, c'est-à-dire des solvants ne détruisant pas la capacité d'échange d'ions de la résine.

Dans un mode de réalisation préférée, on utilise l'eau.

Par résine de type acide, on entend, comme on l'a déjà indiqué, de préférence une résine sulfonique.

Par sel de 7-bromo 6-chlorofébrifugine avec un acide, on entend de préférence un bromhydrate ou un chlorhydrate.

L'invention a particulièrement pour objet un procédé, caractérisé en ce que l'on soumet une résine acide dont les groupements acides sont salifiés par un métal alcalin, à l'action d'un sel acide de 7-bromo 6-chlorofébrifugine, pour obtenir un sel mixte de métal alcalin et de 7-bromo 6-chlorofébrifugine de la résine acide, que l'on soumet, si désiré, à l'action d'un sel soluble de métal alcalino-terreux, pour obtenir un sel mixte de la 7-bromo 6-chlorofébrifugine et de métal alcalino-terreux.

L'invention a tout particulièrement pour objet un procédé caractérisé en ce que l'on soumet une résine sulfonique dont les groupements sulfoniques sont salifiés par du sodium, à l'action du bromhydrate de 7-bromo 6-chlorofébrifugine, pour obtenir un sel mixte de sodium et de 7-bromo 6-chlorofébrifugine de la résine acide, que l'on soumet, si désiré, à l'action du chlorure de calcium, pour obtenir un sel mixte de calcium et de 7-bromo 6-chlorofébrifugine de la résine acide.

Les exemples suivants illustrent l'invention sans toutefois la limiter.

*Exemple 1:*

*Sel mixte de sodium et de 7-bromo 6-chlorofébrifugine de résine sulfonique*

On agite à 20-25°C pendant 16 h un mélange renfermant 100 g de résine Amberlite IRP 69$^{R}$(Na$^+$), 1 l d'eau et 10 g de bromohydrate de 7-bromo 6-chlorofébrifugine racémique micro-nisé. On essore, lave à l'eau et sèche. On obtient un produit titrant 8,8% en poids de 7-bromo 6-chlorofébrifugine (sel I).

*Exemple 2:*

*Sel mixte de calcium et de 7-bromo 6-chlorofébrifugine de résine sulfonique*

Dans 500 ml d'eau distillée, on agite pendant 20 h à 20-25°C un mélange renfermant 20 g de produit préparé à l'exemple 1 et 20 g de chlorure de calcium. On filtre, lave à l'eau jusqu'à disparition d'halogène et sèche. On obtient 20 g de produit titrant 7,9% en poids de 7-bromo 6-chlorofébrifugine (sel II).

*Exemple 3:*

*Sel mixte de calcium et de 7-bromo 6-chlorofébrifugine de résine sulfonique*

*Stade A: Sel mixte de sodium et de 7-bromo 6-chlorofébrifugine de résine sulfonique*

On agite pendant 16 h à 20°C 35 g de résine Amberlite IRP 69$^{R}$(Na$^+$), 10 g de bromhydrate de 7-bromo 6-chlorofébrifugine et 1 l d'eau. On filtre, lave à l'eau et sèche.

On obtient 32 g de sel mixte de sodium et de 7-bromo 6-chlorofébrifugine.

*Stade B: Sel mixte de calcium et de 7-bromo 6-chlorofébrifugine de résine sulfonique*

On agite pendant 23 h un mélange renfermant 32 g de chlorure de calcium, 700 cm³ d'eau distillée et 32 g de résine préparée au stade A. On filtre, lave à l'eau et sèche.

On obtient ainsi 32,1 g de résine titrant 19,8% en poids de 7-bromo 6-chlorofébrifugine (sel III).

*Exemple 4:*

*Sel mixte de calcium et de 7-bromo 6-chlorofébrifugine de résine sulfonique*

En opérant comme à l'exemple 3 à partir de 100 g de résine Amberlite IRP 69$^{R}$(Na$^+$), 1 l d'eau distillée, 10 g de bromhydrate de 7-bromo 6-chlorofébrifugine et 100 g de chlorure de calcium, on obtient une résine titrant 8,4% en poids de 7-bromo 6-chlorofébrifugine (sel IV).

*Exemple 5:*

*Sel mixte de calcium et de 7-bromo 6-chlorofébrifugine de résine sulfonique*

En opérant comme à l'exemple 3 à partir de 50 g de résine Amberlite IRP 69ᵡ(Na⁺), 1000 cm³ d'eau distillée, 10 g de bromhydrate de 7-bromo 6-chlorofébrifugine et 50 g de chlorure de calcium, on obtient une résine titrant 16% en poids de 7-bromo 6-chlorofébrifugine (sel V).

*Exemple 6:*

*Sel mixte de sodium et de 7-bromo 6-chlorofébrifugine de résine sulfonique*

On ajoute 2,3 g de résine cationique CS 120 (Na⁺) (composé sulfonique copolymère styrène-divinylbenzène contenant 14% de divinylbenzène et ayant une capacité d'échange de 4 mg d'équivalent par gramme de résine) dans 61 cm³ d'une solution aqueuse à 0,27% de bromhydrate de 7-bromo 6-chlorofébrifugine et agite pendant 3 h à température ambiante. On filtre le sel obtenu, le lave à l'eau et le sèche. On obtient 2,2 g de produit attendu. La proportion de 7-bromo 6-chlorofébrifugine contenue dans le sel obtenu (sel VI) est de 5,99%.

*Exemple 7:*

*Sel mixte de sodium et de 7-bromo 6-chlorofébrifugine de résine sulfonique*

On injecte dans une colonne échangeuse d'ions 5 g de résine échangeuse d'ions AXT 1Sᵡ(Na⁺) (composé sulfonique copolymère styrènedivinylbenzène, type poreux contenant 20% de divinylbenzène et ayant une capacité d'échange de 0,4 mg d'équivalent par gramme de résine) et fait passer 3 fois une solution de 102 cm³ d'une solution aqueuse à 0,27% de chlorhydrate de 7-bromo 6-chlorofébrifugine. On obtient par filtration le sel formé (sel VII). On le lave à l'eau et le sèche. On obtient 5 g de produit sec. La proportion de 7-bromo 6-chlorofébrifugine dans le sel obtenu est de 4,6%.

Le sel VII peut également être obtenu de la manière suivante:

On ajoute 15 g de résine telle que mentionnée ci-dessus dans 230 cm³ d'une solution méthanolique à 0,3% de 7-bromo 6-chlorofébrifugine. On agite pendant 5 h à température ambiante, puis filtre, lave et sèche le produit comme décrit ci-dessus. On obtient 15 g de produit sec, renfermant 4,6% de 7-bromo 6-chlorofébrifugine.

*Exemple 8:*

*Sel mixte de sodium et de 7-bromo 6-chlorofébrifugine d'une résine carboxylique*

On ajoute 11 g de résine échangeuse de cations Amberlite IRP 88ᵡ(Na⁺) (copolymère acide méthacrylique/divinylbenzène contenant 15% de divinylbenzène et ayant une capacité d'échange de 4,4 mg d'une solution aqueuse à 0,2% de bromhydrate de 7-bromo 6-chlorofébrifugine. On mélange pendant 4 h à température ambiante, filtre, lave et sèche. On obtient 11 g de sel sec (sel VIII) renfermant 6,08% de 7-bromo 6-chlorofébrifugine.

*Exemples 9 à 12:*

En utilisant les mêmes procédés que ci-dessus à partir de quantités variables de 7-bromo 6-chlorofébrifugine ou de son bromhydrate et des résines mentionnées dans le tableau ci-dessous, on obtient les sels correspondants:

| Exemples | Sel | Résine échangeuse de cations utilisée | Procédé utilisé | % de 7-bromo-6-chlorofébrifugine |
|---|---|---|---|---|
| 9 | IX | Résine sulfonique CG 120 (capacité d'échange: 4,4 mg/g) | mélange | 5,46 |
| 10 | X | Résine sulfonique AXT 1S (capacité d'échange: 0,4 mg/g) | colonne | 6,24 |
| 11 | XI | Résine carboxylique Amberlite IRP 88ᵡ (capacité d'échange: 4,4 mg/g) | mélange | 5,15 |
| 12 | XII | Résine sulfonique CG 120 (capacité d'échange: 4,4 mg/g) | mélange | 7,12 |

*Exemple 13:*

*Etude de l'activité coccidiostatique du produit préparé à l'exemple 1.*

On utilise des poulets âgés de 4 semaines. On les répartit en groupes de 20 animaux. La veille du jour de l'infestation, on donne aux poulets une ration alimentaire contenant ou ne contenant pas de produit de l'exemple 1; le jour J, on infeste les poulets avec des coccidies *Eimeria tenella* souche sensible. Un groupe témoin n'est pas infesté.

Le jour J + 7, les animaux sont pesés et sacrifiés. On prélève les cæca, on les ouvre, on les lave et on note les lésions en tenant compte des contenus cæcaux (consistance, présence ou non d'hémorragies) selon le barème suivant:

*Note 0:* pas de lésion: cæca normaux.

*Note 1:* présence de pétéchies sur la muqueuse cæcale.

*Note 2:* muqueuse cæcale hémorragique, légèrement épaissie, cæca de taille normale.

*Note 3:* muqueuse cæcale hémorragique, épaissie, cæca raccourcis.

Les résultats sont les suivants:

| | Témoins sains | Témoins contaminés | Poulets ayant reçu le produit étudié à la dose de 43 mg/kg |
|---|---|---|---|
| note de lésion | 0 | 2,7 | 0,05 |

*Conclusion:* Le produit étudié est très actif contre la coccidiose: au bout d'une semaine de traitement, les animaux infestés sont revenus à l'état normal.

*Exemple 14:*

*Etude de l'activité antileucocytozoonose des produits selon l'invention*

*1) Protocole du test:*

*a) animal utilisé:* jeunes coqs de race White Leghorn âgés de 50 d,

*b) infestation expérimentale:* on administre oralement 300 unités par animal d'oocytes sporulés *Leucocytozoon caulleryi* directement dans le jabot,

*c) produits testés:* on effectue 5 tests comme décrit dans le tableau ci-dessous,

*d) doses de la matière active dans les aliments:* on prépare pour les animaux de la nourriture ne contenant aucun agent préventif contre la leucocytozoonose et ajoute des quantités déterminées de matière active; ces quantités exprimées en ppm figurent dans le tableau ci-dessous,

*e) appréciation du résultat:* entre le début du test (administration du produit et infestation) et la fin (24 d après l'infestation), on observe la parasitémie obtenue et la quantifie en déterminant la quantité d'anticorps dans le sérum, par le test de précipitation sur gel d'agar-agar.

*2) Résultats:*

On détermine l'efficacité comme agents préventifs contre la leucocytozoonose de 5 produits. Les résultats figurent dans le tableau ci-dessous.

| Produit utilisé | Dose (ppm) | Nombre d'animaux | % de prévention de la parasitémie | % d'essais positifs au test de précipitation |
|---|---|---|---|---|
| Sel VIII | 54,6 | 20 | 1,0 | 1,5 |
| Sel IX | 45,9 | 20 | 0 | 0 |
| Sel X | 40,2 | 20 | 0,5 | 1,0 |
| Sel XI | 48,7 | 20 | 0 | 0 |
| Sel XII | 35,2 | 20 | 0,5 | 0,5 |
| Zorin 25 (témoin) | 125 | 20 | 4,0 | 4,5 |

*Exemple 15:*

*Etude de la toxicité*

*a)* La toxicité aiguë par voie orale du produit de l'exemple 1 a été déterminée chez des souris mâles et femelles selon la méthode de Finney.

Les résultats exprimés en $DL_{50}$ (la $DL_{50}$ étant la dose qui provoque la mortalité de 50% des animaux) ont été les suivants:

Souris mâles
  Produit de l'exemple 1     $DL_{50}$ = 66 mg/kg
Souris femelles
  Produit de l'exemple 1     $DL_{50}$ = 57 mg/kg

*Conclusion:* Le produit étudié présente une faible toxicité.

*b)* On a déterminé par ailleurs, par la méthode Litchfield-Wilcoxon, la toxicité aiguë sur souris mâles des sels VI, VIII, IX, XI et XII.

Les résultats sont les suivants:

| Sel testé | $DL_{50}$ (mg/kg) |
|---|---|
| VI | 63,0 |
| VIII | 60,8 |
| IX | 65,3 |
| XI | 67,6 |
| XII | 54,7 |

*Conclusion:* Les produits étudiés présentent une faible toxicité.

*Exemple 16:*

*Exemples de compositions pharmaceutiques*

Produit de l'exemple 1 ................................. 80 g
Excipient à base de carbonate
    de chaux q.s.p. ..........................................1000 g

Cette composition est à incorporer à raison de 500 g par tonne d'aliment complet.

## Revendications

1. Les résines échangeuses d'ions du type acide dont les groupements acides sont au moins partiellement salifiés par la 7-bromo 6-chlorofébrifugine, dextrogyre ou racémique.

2. Les résines telles que définies à la revendication 1, caractérisées en ce que les résines acides utilisées sont des résines sulfoniques.

3. Les résines telles que définies à la revendication 1 ou 2, caractérisées en ce que les résines utilisées sont des composés polyvinylaryliques.

4. Les résines telles que définies à l'une des revendications 1 à 3, caractérisées en ce que les groupements acides ne sont que partiellement salifiés par la 7-bromo 6-chlorofébrifugine.

5. Les résines telles que définies à la revendication 4, caractérisées en ce que les groupements acides non salifiés par la 7-bromo 6-chlorofébrifugine sont salifiés par un métal compatible avec les impératifs de la nutrition animale.

6. Les résines telles que définies à la revendication 5, caractérisées en ce que les groupements acides non salifiés par la 7-bromo 6-chlorofébrifugine sont salifiés par un métal alcalin ou alcalino-terreux.

7. Les résines telles que définies à la revendication 5, caractérisées en ce que les groupements acides non salifiés par la 7-bromo 6-chlorofébrifugine sont salifiés par du manganèse, du magnésium, du fer, du cuivre ou du zinc.

8. Les résines telles que définies à la revendication 6, caractérisées en ce que les groupements acides non salifiés par la 7-bromo 6-chlorofébrifugine sont salifiés par du sodium.

9. Les résines telles que définies à la revendication 6, caractérisées en ce que les groupements acides non salifiés par la 7-bromo 6-chlorofébrifugine sont salifiés par du calcium.

10. Les résines telles que définies à l'une des revendications 4 à 9, caractérisées en ce qu'elles renferment de 1 à 20% et de préférence de 5 à 10% en poids de 7-bromo 6-chlorofébrifugine.

11. Les résines telles que définies à l'une des revendications 1 à 10, caractérisées en ce que la 7-bromo 6-chlorofébrifugine utilisée est le produit racémique.

12. A titre de médicaments, les résines telles que définies à l'une des revendications 1 à 11.

13. Les compositions alimentaires renfermant au moins une des résines définies à l'une des revendications 1 à 11, associée à un mélange nutritif adapté à l'alimentation animale.

14. Procédé de préparation des résines définies à l'une des revendications 1 à 11, caractérisé en ce que l'on salifie au moins partiellement une résine acide par la 7-bromo 6-chlorofébrifugine et obtient ainsi le produit recherché.

15. Procédé de préparation tel que défini à la revendication 14, caractérisé en ce que l'on soumet une résine acide dont les groupements acides sont salifiés par un métal alcalin, à l'action d'un sel acide de 7-bromo 6-chlorofébrifugine, pour obtenir un sel mixte de métal alcalin et de 7-bromo 6-chlorofébrifugine de la résine acide, que l'on soumet, si désiré, à l'action d'un sel soluble de métal non alcalin, pour obtenir un sel mixte de la 7-bromo 6-chlorofébrifugine et de métal non alcalin de la résine acide.

16. Procédé de préparation tel que défini à la revendication 15, caractérisé en ce que l'on soumet une résine acide dont les groupements acides sont salifiés par un métal alcalin à l'action d'un sel acide de 7-bromo 6-chlorofébrifugine pour obtenir un sel mixte de métal alcalin et de 7-bromo 6-chlorofébrifugine de la résine acide, que l'on soumet, si désiré, à l'action d'un sel soluble de métal alcalino-terreux, pour obtenir un sel mixte de la 7-bromo 6-chlorofébrifugine et de métal alcalino-terreux.

17. Procédé de préparation tel que défini à la revendication 16, caractérisé en ce que l'on soumet une résine sulfonique dont les groupements sulfoniques sont salifiés par du sodium, à l'action du bromhydrate de 7-bromo 6-chlorofébrifugine, pour obtenir un sel mixte de sodium et de 7-bromo 6-chlorofébrifugine de la résine acide, que l'on soumet, si désiré, à l'action du chlorure de calcium, pour obtenir un sel mixte de calcium et 7-bromo 6-chlorofébrifugine de la résine acide.

## Patentansprüche

1. Ionenaustauscherharze vom sauren Typ, deren saure Gruppen zumindest teilweise einer Salzbildung mit rechtsdrehendem oder racemischem 7-Brom-6-chlorfebrifugin unterzogen worden sind.

2. Harze gemäss Anspruch 1, dadurch gekennzeichnet, dass die verwendeten sauren Harze Sulfonsäureharze bzw. Sulfoharze sind.

3. Harze gemäss einem der beiden Ansprüche 1 oder 2, dadurch gekennzeichnet, dass die verwendeten Harze Polyvinylarylverbindungen sind.

4. Harze gemäss einem der Ansprüche 1 bis 3, dadurch gekennzeichnet, dass die sauren Gruppen lediglich teilweise einer Salzbildung mit 7-Brom-6-chlorfebrifugin unterzogen worden sind.

5. Harze gemäss Anspruch 4, dadurch gekennzeichnet, dass die sauren Gruppen, die keiner Salzbildung mit 7-Brom-6-chlorfebrifugin unterzogen worden sind, einer Salzbildung mit einem Metall unterzogen worden sind, das mit den Anforderungen für die tierische Ernährung verträglich ist.

6. Harze gemäss Anspruch 5, dadurch gekennzeichnet, dass die sauren Gruppen, die keiner Salzbildung mit 7-Brom-6-chlorfebrifugin unterzogen worden sind, einer Salzbildung mit einem Alkali- oder Erdalkalimetall unterzogen worden sind.

7. Harze gemäss Anspruch 5, dadurch gekennzeichnet, dass die sauren Gruppen, die keiner Salzbildung mit dem 7-Brom-6-chlorfebrifugin unterzogen worden sind, einer Salzbildung mit Mangan, Magnesium, Eisen, Kupfer oder Zink unterzogen worden sind.

8. Harze gemäss Anspruch 6, dadurch gekennzeichnet, dass die sauren Gruppen, die keiner Salzbildung mit 7-Brom-6-chlorfebrifugin unterzogen worden sind, einer Salzbildung mit Natrium unterzogen worden sind.

11 **0 027 768** 12

9. Harze gemäss Anspruch 6, dadurch gekennzeichnet, dass die sauren Gruppen, die keiner Salzbildung mit 7-Brom-6-chlorfebrifugin unterzogen worden sind, einer Salzbildung mit Calcium unterzogen worden sind.

10. Harze gemäss einem der Ansprüche 4 bis 9, dadurch gekennzeichnet, dass sie 1 bis 20%, vorzugsweise 5 bis 10%, bezogen auf das Gewicht, an 7-Brom-6-chlorfebrifugin umfassen.

11. Harze gemäss einem der Ansprüche 1 bis 10, dadurch gekennzeichnet, dass das verwendete 7-Brom-6-chlorfebrifugin das racemische Produkt ist.

12. Als Arzneimittel, Harze gemäss einem der Ansprüche 1 bis 11.

13. Nahrungsmittelzusammensetzungen, enthaltend zumindest eines der Harze gemäss einem der Ansprüche 1 bis 11 zusammen mit einem der tierischen Ernährung angepassten Nährgemisch.

14. Verfahren zur Herstellung von Harzen gemäss einem der Ansprüche 1 bis 11, dadurch gekennzeichnet, dass man zumindest teilweise ein saures Harz einer Salzbildung mit 7-Brom-6-chlorfebrifugin unterzieht und so das gewünschte Produkt erhält.

15. Verfahren gemäss Anspruch 14, dadurch gekennzeichnet, dass man ein saures Harz, dessen saure Gruppen einer Salzbildung mit einem Alkalimetall unterzogen worden sind, der Einwirkung eines sauren Salzes von 7-Brom-6-chlorfebrifugin unterzieht, um ein gemischtes Alkalimetall-7-brom-6-chlorfebrifuginsalz des sauren Harzes zu erhalten, welches man gewünschtenfalls der Einwirkung eines löslichen Nichtalkalimetallsalzes unterzieht, um ein gemischtes 7-Brom-6-chlorfebrifuginnichtalkalimetallsalz des sauren Harzes zu erhalten.

16. Verfahren gemäss Anspruch 15, dadurch gekennzeichnet, dass man ein saures Harz, dessen saure Gruppen einer Salzbildung mit einem Alkalimetall unterzogen worden sind, der Einwirkung eines sauren Salzes von 7-Brom-6-chlorfebrifugin unterzieht, um ein gemischtes Alkalimetall-7-brom-6-chlorfebrifuginsalz des sauren Harzes zu erhalten, welches man gewünschtenfalls der Einwirkung eines löslichen Erdalkalimetallsalzes unterzieht, um ein gemischtes 7-Brom-6-chlorfebrifuginerdalkalimetallsalz zu erhalten.

17. Verfahren gemäss Anspruch 16, dadurch gekennzeichnet, dass man ein Sulfonsäureharz bzw. Sulfoharz, dessen Sulfonsäuregruppen einer Salzbildung mit Natrium unterzogen worden sind, der Einwirkung von 7-Brom-6-chlorfebrifuginhydrobromid unterzieht, um ein gemischtes Natrium-7-brom-6-chlorfebrifuginsalz des sauren Harzes zu erhalten, welches man gewünschtenfalls der Einwirkung von Calciumchlorid unterzieht, um ein gemischtes Calcium-7-brom-6-chlorfebrifuginsalz des sauren Harzes zu erhalten.

**Claims**

1. Ion exchange resins of acid type of which the acid groups are at least partially salified by 7-bromo 6-chlorofebrifugine, dextro-rotatory or racemic.

2. Resins as defined in claim 1, characterized in that the acid resins utilized are sulphonic resins.

3. Resins as defined in claim 1 or 2, characterized in that the resins utilized are polyvinylaryl compounds.

4. Resins as defined in any one of the claims 1 to 3, characterized in that the acid groups are only partially salified by 7-bromo 6-chlorofebrifugine.

5. Resins as defined in claim 4, characterized in that the acid groups not salified by 7-bromo 6-chlorofebrifugine are salified by a metal compatible with the demands of animal nutrition.

6. Resins as defined in claim 5, characterized in that the acid groups not salified by 7-bromo 6-chlorofebrifugine are salified by an alkali metal or an alkaline-earth metal.

7. Resins as defined in claim 5, characterized in that the acid groups not salified by 7-bromo 6-chlorofebrifugine are salified by manganese, magnesium, iron, copper or zinc.

8. Resins as defined in claim 6, characterized in that the acid groups not salified by 7-bromo 6-chlorofebrifugine are salified by sodium.

9. Resins as defined in claim 6, characterized in that the acid groups not salified by 7-bromo 6-chlorofebrifugine are salified by calcium.

10. Resins as defined by any one of the claims 4 to 9, characterized in that they contain from 1 to 20% and preferably from 5 to 10% by weight of 7-bromo 6-chlorofebrifugine.

11. Resins as defined in any one of the claims 1 to 10, characterized in that the 7-bromo 6-chlorofebrifugine utilized is the racemic product.

12. As medicaments, the resins as defined in any one of the claims 1 to 11.

13. Alimentary compositions containing at least one of the resins defined in any one of the claims 1 to 11, associated with a nutritive mixture suitable for animal alimentation.

14. Process for the preparation of the resins defined in any one of the claims 1 to 11, characterized in that an acid resin is salified at least partially by 7-bromo 6-chlorofebrifugine and the product sought is so obtained.

15. Process for the preparation as defined in claim 14, characterized in that an acid resin of which the acid groups are salified by an alkali metal is submitted to the action of an acid salt of 7-bromo 6-chlorofebrifugine, in order to obtain an alkali metal and 7-bromo 6-chlorofebrifugine mixed salt of the acid resin, which, if desired, is submitted to the action of a soluble salt of a non-alkali metal, so as to obtain a 7-bromo 6-chlorofebrifugine and non-alkali metal mixed salt of the acid resin.

16. Process for the preparation as defined in claim 15, characterized in that an acid resin of which the acid groups are salified by an alkali metal is submitted to the action of an acid salt of 7-bromo 6-chlorofebrifugine so as to obtain an alkali metal and 7-bromo 6-chlorofebrifugine mixed salt of the acid resin, which, if desired, is submitted to the action of a soluble salt of alkaline-earth metal, so as to obtain a 7-bromo 6-chlorofebrifugine and alkaline-earth metal mixed salt.

17. Process for the preparation as defined in claim 16, characterized in that a sulphonic resin of which the sulphonic groups are salified by sodium is submitted to the action of the hydrobromide of 7-bromo 6-chlorofebrifugine, so as to obtain a sodium and 7-bromo 6-chlorofebrifugine mixed salt of the acid resin, which, if desired, is submitted to the action of calcium chloride, so as to obtain a calcium and 7-bromo 6-chlorofebrifugine mixed salt of the acid resin.